Europäisches Patentamt

European Patent Office

Office européen des brevets

(19)

Publication number: **0 284 330**
**A1**

# EUROPEAN PATENT APPLICATION

(21) Application number: 88302468.9

(51) Int. Cl.4: **A61B 5/00**

(22) Date of filing: **22.03.88**

(30) Priority: **23.03.87 US 28991**

(43) Date of publication of application:
**28.09.88 Bulletin 88/39**

(34) Designated Contracting States:
**DE FR GB**

(71) Applicant: **EASTMAN KODAK COMPANY**
**Patent Department 343 State Street**
**Rochester New York 14650(US)**

(72) Inventor: **Duarte, Francisco Javier Eastman**
**Kodak Company**
**Patent Department 343 State Street**
**Rochester New York 14650(US)**

(74) Representative: **Davis, Ian Ellison et al**
**Kodak Limited Patent Department Headstone**
**Drive**
**Harrow Middlesex HA1 4TY(GB)**

(54) **Two-laser therapy and diagnosis device.**

(57) There is disclosed a device (10) that permits two lasers (20, 30) to perform the complete functions of diagnosis and treatment in photoradiation therapy. The treatment laser (30) is a dye laser that requires a pumping laser, and a third laser is omitted by constructing the diagnosis laser (20) to operate alternatively as the pumping source for the treatment laser or to diagnose. Means (28) are provided to alternatively direct the diagnosis laser (20) onto either the treatment laser (30) or the patient (P).

EP 0 284 330 A1

## TWO-LASER THERAPY AND DIAGNOSIS DEVICE

This invention relates to a device for diagnosing and treating cancer with photoradiation therapy.

In the treatment of cancer, one method that is used is photoradiation therapy in which a dye, such as a hematoporphyrin derivative, is absorbed, usually selectively, by cancer cells. This type of dye can also be used in diagnosis by exciting the dye at short wavelengths, for example 405 nm, to cause it to fluoresce at longer wavelengths, in the 620-720 nm range. The fluorescence is used to detect the location of the cancer. After locating the tumor cells from their fluorescene, a second laser emitting at about 630 nm can then be used, since that is an appropriate absorbance wavelength for such hematoporphyrin derivatives. When the derivative is exposed by the second laser, a singlet oxygen emission occurs and this emission is fatal to the cancer cell by which the dye is absorbed. It is also known that one or both of the aforementioned lasers can be pumped or stimulated by a third laser, which can be an excimer laser. Since either of the two lasers used for diagnosis and treatment often in fact requires a laser as the pump or stimulus, there is thus a requirement for 3 or 4 lasers in all.

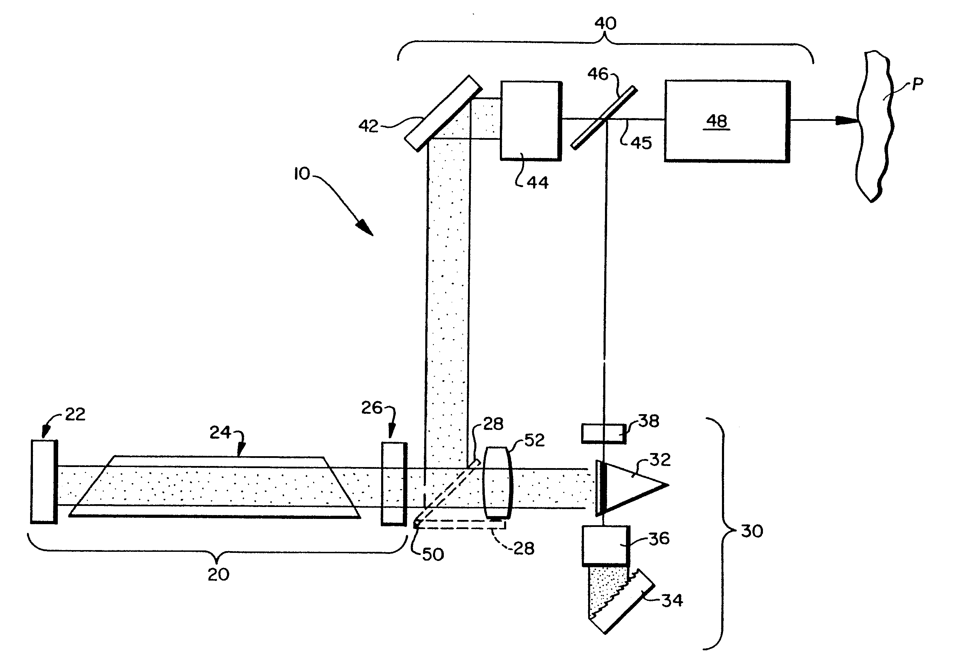

Such a system is shown, for example, in U. S. Patent No. 4,556,057, wherein the same excimer laser is used to pump or stimulate either the diagnosis laser or the treatment laser. Figure 1 of that patent illustrates that the same fiber optics and endoscope carries first the diagnosis laser beam, and upon confirmation of the locus of the tumor, it carries then the treatment laser beam.

Therefore, prior to this invention a problem has occurred in the number of lasers required. A system that requires fewer lasers would not only simplify the procedure, but it would also reduce the cost of the equipment.

In accord with one aspect of the invention, such problems as are noted above have been addressed by a device for diagnosing and treating cancer, the device comprising a diagnosis laser and a treatment laser, each operating at a different preferred frequency, and means for pumping at least one of the lasers. The device is characterized in that there is included means for selectively directing the output of the diagnosis laser alternatively onto a selected portion of the patient, or into the treatment laser, so that the diagnosis laser is also a pumping means for the treatment laser.

Thus, it is an advantageous feature of the invention that only two lasers need to be used for the entire photoradiation therapy, even when one of the lasers needs a pumping laser for stimulation.

It is a related advantageous feature of the invention that the equipment needed for the photoradiation therapy is simplified.

The present invention will now be described by way of example with reference to the accompanying drawings in which:

The Figure is a partially schematic illustration of a device constructed in accordance with the invention.

The preferred embodiment features the use of a recombination laser as the diagnosis laser. In addition, the invention is useful when the laser used alternatively for diagnosis or pumping, is some other laser, such as a copper vapor laser, provided it emits light at the desired wavelengths.

As shown in the Figure of the drawings, the device 10 comprises a first laser system 20, a second laser system 30, and optics 40 used to deliver the beams of the lasers onto a selected portion of a patient P. Laser system 20 comprises a total reflector 22 for the wavelengths emitted by laser 24 of the first laser system, and a partial reflector output coupler 26 for the wavelengths selected. Preferably, laser 24 is a recombination laser which is readily obtained from a conventional copper laser by replacing the Cu with either Sr or Ca, and modifying the mirror at 22 so as to be totally reflective at either 430 nm or 373.7 nm, respectively. (Coupler 26 is modified accordingly.) Examples of recombination lasers are included in IEEE J. of Quantum Electronics Vol. QE-21, p. 1563-66 (1985).

The output of laser system 20 is ideally suited for diagnosis of cancer cells by exciting a hematoporphyrin derivative absorbed by the cells in patient P. To this end, mirror 28 is disposed to reflect the light emitted by laser system 20 to the optics 40. Such optics preferably comprise another total reflector 42 that is substantially identical to reflector 22, a telescope 44 to constrict the beam from reflector 42 to a fine beam 45 of very narrow width, and a beam splitter 46 whose function will become more apparent hereinafter. Optionally, fiber optics 48 are useful to carry the beam 45 to a precise location on the body of patient P, as is well-known.

A second laser system 30 is also provided, preferably comprising dye cell 32, grating 34, multiple-prism beam expander 36, and a partial reflector output coupler 38 for 630 nm wavelengths. That is, laser system 30 is preferably a dye laser, known to be suitable for treatment of cancer cells that have absorbed the hematoporphyrin derivative.

Laser system 30 requires a pumping source, and in accordance with one aspect of the invention, laser system 20 is adapted to be used alternately for this purpose. To permit this, mirror 28 is swivable about pivot 50, and can be moved by hand or by suitable automatic means, not shown. A cylindrical lens 52 preferably is imposed between cell 32 and coupler 26.

Any conventional dye laser is useful as system 30, for example, those described in Applied Optics, Vol. 23, p. 1391 ff (1984). Useful dyes for such a laser include known dyes Rhodamine 590 or 610, available for example from Exciton. Alternatively, a laser other than a dye laser, which also requires stimulation, can be used. One example is an optically pumped $Te_2$ laser excited at 430.5 nm and emitting in the 623.93-641.66 nm range as described in Soviet Journal of Quantum Electronics Vol. 15, p. 288 (1985). (The treatment frequencies must be primarily in the red wavelengths, whereas diagnosis frequencies use primarily blue wavelengths.) Still further, laser system 30 can be rendered broad-band, if desired, by converting grating 34 and expander 36 into a total reflector, as is well-known.

In operation, a dye such as bis-1-[8-(1-hydroxyethyl) porphyrin-yl]ethyl ether is absorbed by the patient, preferentially at the tumor site. To find that site, mirror 28 is placed in position to intercept the beam outputted by coupler 26, so that laser system 20 acts as a diagnosis laser first. Then, mirror 28 is moved out of the way, once the cancer is precisely located, so that laser system 20 acts to pump laser system 30 into a lasing condition, thereby treating the cancer cell to 630 nm wavelength coherent light.

Alternatively, other forms of reflectors are useful at 28, for example a mirror that slides in and out of its reflecting position that intercepts that output from coupler 26.

## Claims

1. A device for diagnosing and treating cancer in a patient, the device comprising a diagnosis laser and a treatment laser, each operating at a different preferred frequency, and means for pumping at least one of said lasers,

characterized in that the device includes means for selectively directing the output of said diagnosis laser alternatively onto a selected portion of a patient, or into said treatment laser,

so that the diagnosis laser is also a pumping means for the treatment laser.

2. A device as defined in claim 1, wherein said treatment laser is constructed to emit primarily in the red wavelengths.

3. A device as defined in claim 1 or 2, wherein said diagnosis laser emits primarily in the blue wavelengths.

4. A device as defined in claim 1, 2 or 3, wherein said treatment laser is a dye laser and said diagnosis laser is a recombination laser.

5. A device as defined in any one of claims 1 to 4, wherein said directing means comprises a pivotable mirror.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-4 336 809 (W.G. CLARK) * column 2, line 14 - column 4, line 20; figures 1,5 * | 1-3 | A 61 B 5/00 |
| A | GB-A-2 125 986 (HAMAMATSU PHOTONICS K.K.) * abstract; page 2, line 35; page 3, line 38; figures 2,6 * | 1-4 | |
| A | EP-A-0 123 548 (W.A. PROVOST) * page 12, line 20 - page 13, line 22; figure 4 * | 5 | |

TECHNICAL FIELDS SEARCHED (Int. Cl.4)

A 61 B 5/00
A 61 B 1/00

The present search report has been drawn up for all claims

| Place of search | Date of completion of the search | Examiner |
|---|---|---|
| BERLIN | 16-06-1988 | WEIHS J.A. |

EPO FORM 1503 03.82 (P0401)